# EUROPEAN PATENT APPLICATION

(11) **EP 3 301 588 A1**
(43) Date of publication of application: **04.04.2018**
(21) Application number: 16191678.8
(22) Date of filing: 30.09.2016
(51) Int. Cl.: G06F 17/30, G06F 19/00

(54) **METHOD AND APPARATUS FOR SUPPLEMENTING AN ARTICLE WITH CONTEXTUALLY-RELEVANT HEALTH DATA**

(71) Applicant: Nokia Technologies Oy, 02610 Espoo (FI)
(72) Inventor: Favretto, Stuart, San Francisco, CA California 94107 (US); Zavattaro, Manuela, San Francisco, CA California 94110 (US)
(74) Representative: Nokia EPO representatives

(57) **Abstract**

A method, apparatus and computer program product are provided to present health data of a user in a more informed context. In relation to a method, an article is identified that provides health-related information and health data of a user is identified that is associated with the health-related information of the article. The method also includes supplementing the article with the health data of the user and causing the article supplemented with the health data of the user to be provided to the user. The resulting article, as supplemented, places the health data in context and better informs the user.

## Description

### TECHNOLOGICAL FIELD

An example embodiment relates generally to the provision of health-related information and, more particularly, to the supplementation of an article that provides health-related information with contextually-relevant health data of a user.

### BACKGROUND

People are consuming increasing amounts of health-related information. People may consume health-related information for various reasons. Some people may simply have an interest in health-related topics, while other people may consume health-related information in an effort to inform their behavior so as to improve their health. Other people may consume health-related information that is pushed by a healthcare provider, a pharmacist or the like for educational, promotional or other purposes.

Although people may read a number of articles that provide health-related information, people may struggle to appreciate the relationship of their health condition to the health-related information conveyed by the article. For example, the article may provide information regarding the desired range of various health-related parameters, but the reader may be unaware of their own corresponding health-related parameters and, as such, be unable to place the health-related information in the context of their own health condition.

In some instances, people may have access to and review health data regarding their health condition. For example, people may review their blood sugar readings or other similar health-related parameters that are collected over the course of time. While this health data provides information regarding the health condition of a person, the person may be unable to conveniently compare their own health data to comparable health data of other people in order to place their own health data in context.

Thus, while the consumption of health-related information is increasing, people may not be readily able to put their own health data in the context of more general health-related information. As such, people may not be as fully informed by the health-related information that they consume as would be desirable.

### BRIEF SUMMARY

A method, apparatus and computer program product are provided in accordance with an example embodiment in order to provide increased context for the health data of a user. In this regard, the method, apparatus and computer program product of an example embodiment may be configured to supplement an article with contextually-relevant health data of the user such that the resulting article, as supplemented, places the health data in context and better informs the user. Thus, the method, apparatus and computer program product of an example embodiment address technical problems related to the efficient and timely identification of contextually-relevant health data in light of the health-related information provided by an article and then supplementing the article with the contextually-related health data.

In an example embodiment, a method is provided that includes identifying an article providing health-related information and identifying health data of a user that is associated with the health-related information of the article. The method also includes supplementing the article with the health data of the user and causing the article supplemented with the health data of the user to be provided to the user.

The method of an example embodiment identifies the health data by identifying the health data of the user based upon data associated with the article that at least partially defines the context of the article. For example, the data associated with the article may include metadata associated with the article. Additionally or alternatively, the method may identify the health data of the user based upon data associated with the article by scraping the article to identify data within the article that at least partially defines a context of the article.

The method of an example embodiment identifies health data by identifying the health data of the user in the response to execution of an instruction included within the article. The method of an example embodiment also includes receiving health data from the user from a device worn by the user and causing the health data of the user to be stored. The method of an example embodiment also includes additionally supplementing the article with empirical data associated with the health-related information of the article. In this regard, the supplementation of the article with the health data of the user and the empirical data may include providing a comparison between the health data of the user and the empirical data within the article. In an example embodiment, the method also includes obtaining the empirical data from the article. The method of an example embodiment identifies an article by analyzing the health data of the user in order to detect a health issue of the user and identifying the article based upon the health issue of the user such that the article provides health-related information relevant to the health issue. The method of an example embodiment also includes supplementing the article with the health data of the user by creating space within the article in which the health data is inserted without eliminating any content associated with the article.

In another example embodiment, an apparatus is provided that includes at least one processor and at least one memory including computer program code with the at least one memory and the computer code configured to, with the processor, cause the apparatus to at least identify an article providing health-related information. The at least one memory and the computer program code are also configured to, with the processor, cause the apparatus to identify health data of a user that is associated with the health-related information of the article and to supplement the article with the health data of the user. The at least one memory and the computer program code are further configured to, with the processor, cause the apparatus to cause the article as supplemented with the health data of the user to be provided to the user.

The at least one memory and the computer program code are configured to, with the processor, cause the apparatus of an example embodiment to identify health data by identifying health data of the user based upon data associated with the article that at least partially defines the context of the article. For example, the data associated the article may include metadata associated with the article. In an example embodiment, the at least one memory and the computer program code are configured to, with the processor, cause the apparatus to identify health data of the user based upon data associated with the article by scraping the article to identify data within the article that at least partially defines the context of the article.

The at least one memory and the computer program code are configured to, with the processor, cause the apparatus of an example embodiment to identify health data by identifying the health data of the user in response to execution of an instruction included within the article. The at least one memory and the computer program code are configured to, with the processor, cause the apparatus of an example embodiment to receive health data of the user from the device worn by the user and to cause the health data of the user to be stored. The at least one memory and the computer program code are configured to, with the processor, cause the apparatus of an example embodiment to additionally supplement the article with empirical data associated with the health-related information of the article. For example, the supplementation of an article with the health data of the user may include the provision of a comparison between the health data of the user and the empirical data within the article. In this regard, the empirical data may be obtained in an example embodiment from the article. The at least one memory and the computer program code are configured to, with the processor, cause the apparatus of an example embodiment to identify an article by analyzing the health data of the user in order to detect a health issue of the user and identifying the article based upon the health issue of the user such that the article provides health-related information relevant to the health issue. The at least one memory and the computer program code are configured to, with the processor, cause the apparatus of an example embodiment to supplement the article with health data of the user by creating space within the article in which the health data is inserted without eliminating any content associated with the article.

In a further example embodiment, a computer program product is provided that includes at least one non-transitory computer-readable storage medium having computer-executable program code instructions stored therein with the computer-executable program code instructions including program code instructions to at least identify an article providing health-related information. The computer-executable program code instructions also include program code instructions to identify health data of a user that is associated with the health-related information of the article and program code instructions to supplement the article with the health data of the user. The computer-executable program code instructions further include program code instructions to cause the article as supplemented with the health data of the user to be provided to the user.

In an example embodiment, the program code instructions to identify health data include program code instructions to identify health data of the user based upon data associated with the article that at least partially defines a context of the article. For example, the data associated with the article may include metadata associated with the article. In an example embodiment, the program code instructions to identify health data of the user based upon data associated with the article may include program code instructions to scrape the article to identify data within the article that at least partially defines a context of the article. The program code instructions to identify health data may include, in an example embodiment, program code instructions to identify the health data of the user in response to execution of an instruction included within the article.

The computer-executable program code instructions of an example embodiment also include program code instructions to receive health data of the user from a device worn by the user and program code instructions to cause the health data of the user to be stored. The computer-executable program code instructions of an example embodiment also include program code instructions to additionally supplement the article with empirical data associated with the health-related information of the article. The program code instructions to supplement the article with the health data of the user and with empirical data may include program code instructions to provide a comparison between the health data of the user and the empirical data within the article. In this example embodiment, the computer-executable program code instructions also include program code instructions to obtain the empirical data from the article. The program code instructions to identify the article may include program code instructions to analyze the health data of the user in order to detect a health issue of the user and program code instructions to identify the article based upon the health issue of the user such that the article provides health-related information relevant to the health issue. The program code instructions to supplement the article with the health data of the user may include program code instructions to create space within the article in which the health data is inserted without eliminating any content associated with the article.

In yet another example embodiment, an apparatus is provided that includes means for identifying an article providing health-related information and means for identifying health data of a user that is associated with the health-related information of the article. The apparatus of this example embodiment also includes means for supplementing the article with the health data of the user and means for causing the article as supplemented with the health data of the user to be provided to the user.

### BRIEF DESCRIPTION OF THE DRAWINGS

Having thus described certain example embodiments of the present disclosure in general terms, reference will hereinafter be made to the accompanying drawings, which are not necessarily drawn to scale, and wherein:
Figure 1 is a representation of an article as supplemented with the health data of a user and as may be presented upon a display in accordance with an example embodiment of the present innovation;
Figure 2 is a representation of another article as supplemented with the health data of a user and as may be presented upon a display in accordance with another example embodiment of the present innovation;
Figure 3 is a block diagram of a system that may support the supplementation of an article with the health data of a user in accordance with an example embodiment of the present innovation;
Figure 4 is a schematic representation of a system that may support the supplementation of an article with the health data of a user in accordance with an example embodiment of the present innovation;
Figure 5 is a block diagram of an apparatus that may be specifically configured in accordance with an example embodiment of the present innovation;
Figure 6 is a flow chart illustrating operations performed, such as by the apparatus of Figure 5, in accordance with an example embodiment of the present innovation in order to supplement an article with the health data of a user; and
Figure 7 is a representation of an article illustrating indications in the form of tags indicating that health data of the user and/or empirical data should be identified for presentation along with the article in accordance with an example embodiment of the present innovation.

### DETAILED DESCRIPTION

Some embodiments will now be described more fully hereinafter with reference to the accompanying drawings, in which some, but not all, embodiments of the innovation are shown. Indeed, various embodiments of the innovation may be embodied in many different forms and should not be construed as limited to the embodiments set forth herein; rather, these embodiments are provided so that this disclosure will satisfy applicable legal requirements. Like reference numerals refer to like elements throughout. As used herein, the terms "data," "content," "information," and similar terms may be used interchangeably to refer to data capable of being transmitted, received and/or stored in accordance with embodiments of the present innovation. Thus, use of any such terms should not be taken to limit the spirit and scope of embodiments of the present innovation.

Additionally, as used herein, the term 'circuitry' refers to (a) hardware-only circuit implementations (e.g., implementations in analog circuitry and/or digital circuitry); (b) combinations of circuits and computer program product(s) comprising software and/or firmware instructions stored on one or more computer readable memories that work together to cause an apparatus to perform one or more functions described herein; and (c) circuits, such as, for example, a microprocessor(s) or a portion of a microprocessor(s), that require software or firmware for operation even if the software or firmware is not physically present. This definition of 'circuitry' applies to all uses of this term herein, including in any claims. As a further example, as used herein, the term 'circuitry' also includes an implementation comprising one or more processors and/or portion(s) thereof and accompanying software and/or firmware. As another example, the term 'circuitry' as used herein also includes, for example, a baseband integrated circuit or applications processor integrated circuit for a mobile phone or a similar integrated circuit in a server, a cellular network device, other network device, and/or other computing device.

As defined herein, a "computer-readable storage medium," which refers to a non-transitory physical storage medium (e.g., volatile or non-volatile memory device), can be differentiated from a "computer-readable transmission medium," which refers to an electromagnetic signal.

A method, apparatus and computer program product are provided in accordance with an example embodiment in order to provide contextually relevant health-related information to a user. In this regard, the method, apparatus and computer program product of an example embodiment may identify an article providing health-related information, such as an article that provides health-related information relevant to a health issue of the user, and may supplement the article with health data of the user, thereby placing the health data of a user in context relative to the health-related information provided by the article. The present disclosure may provide a technical effect of increasing contextually relevant data in view of biometric health data of a particular user. More specifically, devices configured to gather personal health data may become more useful when the gathered data is presented in contextually relevant media.

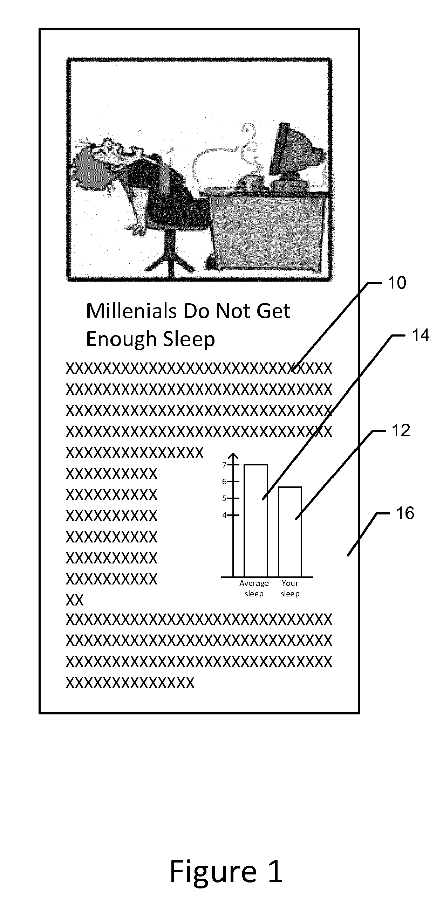

By way of example, Figure 1 depicts an article 10 providing health-related information. In this example, the article is entitled "Millennials Do Not Get Enough Sleep" and, as such, provides the results of a sleep study of millennials who, on average, sleep less than the recommended daily amount. As described below, this article 10 may have been selected as a result of a health issue of the user, such as chronic fatigue, insomnia or a more general pattern of sleeping less than is desired. In accordance with an example embodiment, the article 10 is supplemented with health data 12 of the user which may, for example, provide an indication of an average length of the user's sleep at night. As a result, the user is able to view the health-related information provided by the article 10 in context relative to their own sleep patterns. Furthermore, the article 10 may, in some embodiments, be also supplemented with empirical data 14 associated with the health-related information of the article, such as by providing health data regarding the average amount that a millennial sleeps at night, such as in comparison to the average amount that the user sleeps at night, so as to provide additional context to the health data 12 of the user. As such, the article 10 not only serves an educational purpose with respect to the health-related information, but also facilitates conclusions drawn by the user regarding their own health data 12 in the context of the health-related information provided by the article and, in some embodiments, in the further context provided by empirical data 14. Thus, the user may be better informed and may choose to make changes to their lifestyle or otherwise address the health issues with which the health data 12 of the user relates once the user has considered their own health data in the context of the health-related information provided by the article 10 and, in some embodiments, in further comparison with empirical data 14.

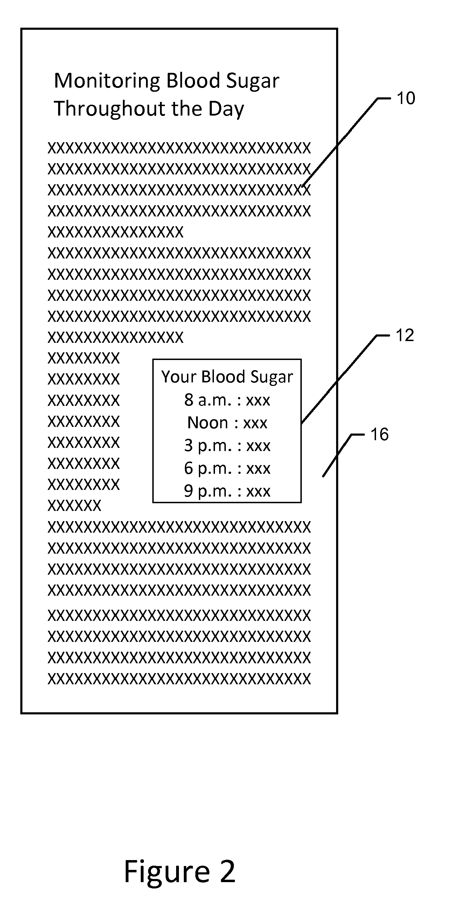

Another example of an article 10 providing health-related information is depicted in Figure 2. The article 10 is entitled "Monitoring Blood Sugar Throughout the Day" and may have been selected as a result of the user suffering from diabetes or another health condition in which blood sugar monitoring is of importance. In accordance with an example embodiment, the article 10 is supplemented with health data 12 of the user which may, for example, provide an indication of the average blood sugar of the users at different times. As a result, the user is able to place the health-related information provided by the article 10 in context relative to their own blood sugar readings. Unlike the article 10 of Figure 1, the health data 12 of the user is not compared to empirical data in graphical form, but nonetheless is presented in the context of the health-related information provided by the article.

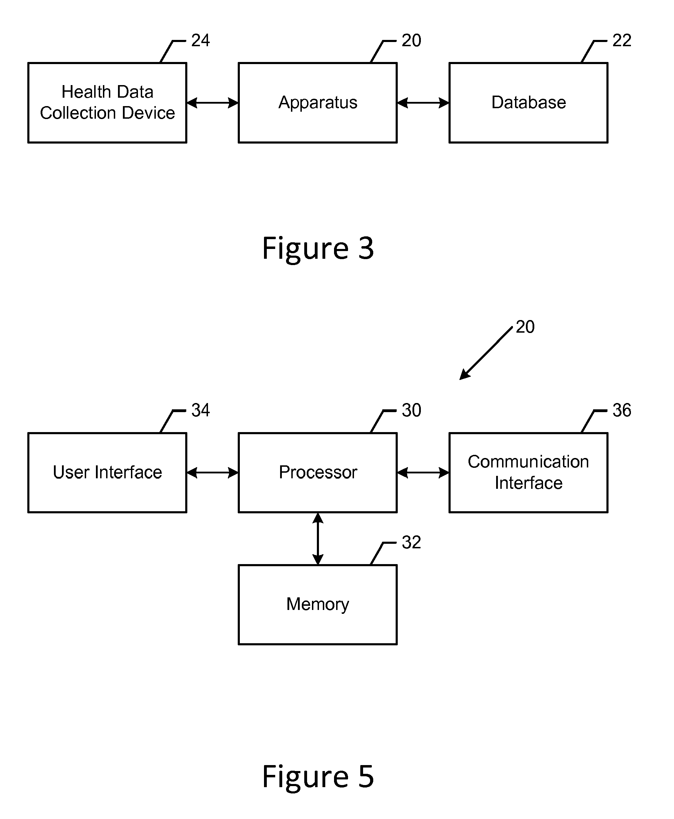

In an example embodiment depicted in Figure 3, an apparatus 20 is provided in order to identify an article 10 providing health-related information and to supplement the article with health data 12 of the user. The apparatus 20 may be embodied by or otherwise associated with a variety of different computing devices including, for example, a mobile terminal, such as a portable digital assistant (PDA), mobile telephone, smartphone, pager, mobile television, gaming device, laptop computer, camera, tablet computer, touch surface, video recorder, radio, electronic book, or any combination of the aforementioned, and other types of voice and text communications systems. Alternatively, the computing device may be a fixed computing device, such as a personal computer, a computer workstation, a server or the like. While the apparatus 20 may be embodied by a single computing device, the apparatus of some example embodiments may be embodied in a distributed manner with some components of the apparatus embodied by different computing devices.

The apparatus 20 may include or, as shown in the embodiment of Figure 3, be in communication with a database 22. The database 22 may store the health data 12 of the user and, in some embodiments, empirical data 14. In this regard, the empirical data 14 includes health data from other people, such as the health data of a larger population, which may or may not include the user. In some instances, the database 22 may also include articles 10 that provide health-related information. In an example embodiment in which the apparatus 20 is in communication with the database 22, the database may be configured in various manners, such as a cloud-based database, a server, a government database, a peer review database, a private database, such as maintained by a health organization, e.g., an insurance company or a health care provider, or the like.

As noted above, the health data 12 of the user may be stored by the database 22. Alternatively, the apparatus 20 may access the health data 12 of the user in other manners. For example, the user may wear, carry or otherwise utilize one or more health data collection devices 24. The health data collection devices 24 collect health data 12 of the user and provide the health data to the apparatus 20, either directly as shown in the embodiment of Figure 3 or indirectly by initially storing the health data, such as in the database 22 or in another memory device for subsequent access by the apparatus. A variety of different health data collection devices 24 may be utilized depending upon the health issues of the user or about which the user and/or a health care provider of the user are interested. For example, the health data collection device 24 may include a blood glucose monitor for providing health data indicative of the blood glucose level of the user at different points in time. Additionally or alternatively, the health data collection device 24 may be a heart monitor for monitoring the heart rate and/or the heart rate variability of the user during various periods of time. As another example, the health data collection device 24 may be a blood pressure cuff for providing health data indicative of the blood pressure of the user at one or more points in time. In some examples, the health data collection device 24, may be a wearable computing device configured to gather biometric information such as heart rate, respiration rate, glucose measurements, eye movements, sleep related data, and the like. The foregoing examples are intended to illustrate several types of health data collection devices 24 and to evidence that the health data collection device may be any of a wide variety of devices configured to collect health data 12 of the user, such as temperature, movement, heart rate, respiration rate, cortisol levels, etc., for analysis as described herein.

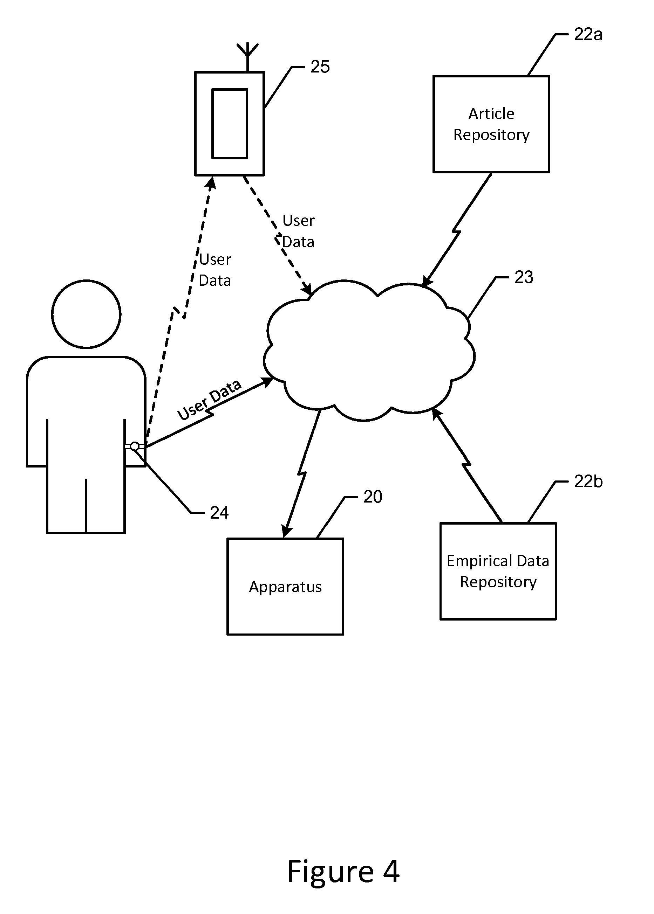

By way of another example, Figure 4 depicts an embodiment in which a user wears a health data collection device 24. In Figure 4, the health data collection device 24 is shown as a watch, but the health data collection device may be differently embodied as, for example, other wearable devices configured to collect health data 12 of the user and to provide the health data to the apparatus 20, the database 22 or the like. The health data collection device 24 may, at least temporarily, store the health data 12 of the user locally. In the embodiment of Figure 4, the health data collection device 24 is configured to provide the user data to a cloud-based database 23. Additionally or alternatively (and as shown in dashed lines in Figure 4), the health data collection device 24 may be configured to provide the health data 12 of the user to a mobile terminal 25, such as a portable digital assistant (PDA), mobile telephone, smartphone, laptop computer, tablet computer or the like, of the user, such as via a local area network or other proximity-based communication technique. The mobile terminal 25 may be configured to store the user data locally and to provide the user data (or a representation or summary thereof) to a database, such as the cloud-based database 23 in the embodiment of Figure 4. The cloud-based database 23 of the embodiment of Figure 4 may also receive other types of information, such as articles 10 from an article repository 22a and/or empirical data 14 from an empirical data repository 22b. The cloud-based database 23 may, in turn, provide an article 10 and the related health data 12 of the user (and optionally related empirical data 14) to the apparatus 20 for presentation to the user. Although the embodiment of Figure 4 depicts the collection of the articles 10, the health data 12 of the user and the empirical data 14 by the cloud-based database 23 and the subsequent provision to the apparatus 20, some or all of the articles, the health data of the user and the empirical data may, instead, be provided directly to the apparatus without collection by the cloud-based database.

As noted above, the apparatus 20 may be embodied by any of a wide variety of different types of computing devices. Regardless of the type of computing device, the apparatus 20 of an example embodiment depicted in Figure 5 is configured to include or otherwise be in communication with a processor 30, a memory device 32 and a user interface 34 and optionally a communication interface 36. In some embodiments, the processor 30 (and/or co-processors or any other processing circuitry assisting or otherwise associated with the processor) may be in communication with the memory device 32 via a bus for passing information among components of the apparatus 20. The memory device 32 may be non-transitory and may include, for example, one or more volatile and/or non-volatile memories. In other words, for example, the memory device 32 may be an electronic storage device (e.g., a computer readable storage medium) comprising gates configured to store data (e.g., bits) that may be retrievable by a machine (e.g., a computing device like the processor). The memory device 32 may be configured to store information, data, content, applications, instructions, or the like for enabling the apparatus 20 to carry out various functions in accordance with an example embodiment of the present innovation. For example, the memory device 32 could be configured to buffer input data for processing by the processor 30. Additionally or alternatively, the memory device 32 could be configured to store instructions for execution by the processor 30.

As described above, the apparatus 20 may be embodied by a computing device. However, in some embodiments, the apparatus 20 may be embodied as a chip or chip set. In other words, the apparatus 20 may comprise one or more physical packages (e.g., chips) including materials, components and/or wires on a structural assembly (e.g., a baseboard). The structural assembly may provide physical strength, conservation of size, and/or limitation of electrical interaction for component circuitry included thereon. The apparatus 20 may therefore, in some cases, be configured to implement an embodiment of the present innovation on a single chip or as a single "system on a chip." As such, in some cases, a chip or chipset may constitute means for performing one or more operations for providing the functionalities described herein.

The processor 30 may be embodied in a number of different ways. For example, the processor 30 may be embodied as one or more of various hardware processing means such as a coprocessor, a microprocessor, a controller, a digital signal processor (DSP), a processing element with or without an accompanying DSP, or various other processing circuitry including integrated circuits such as, for example, an ASIC (application specific integrated circuit), an FPGA (field programmable gate array), a microcontroller unit (MCU), a hardware accelerator, a special-purpose computer chip, or the like. As such, in some embodiments, the processor 30 may include one or more processing cores configured to perform independently. A multi-core processor 30 may enable multiprocessing within a single physical package. Additionally or alternatively, the processor 30 may include one or more processors configured in tandem via the bus to enable independent execution of instructions, pipelining and/or multithreading.

In an example embodiment, the processor 30 may be configured to execute instructions stored in the memory device 32 or otherwise accessible to the processor. Alternatively or additionally, the processor 30 may be configured to execute hard coded functionality. As such, whether configured by hardware or software methods, or by a combination thereof, the processor 30 may represent an entity (e.g., physically embodied in circuitry) capable of performing operations according to an embodiment of the present innovation while configured accordingly. Thus, for example, when the processor 30 is embodied as an ASIC, FPGA or the like, the processor may be specifically configured hardware for conducting the operations described herein. Alternatively, as another example, when the processor 30 is embodied as an executor of software instructions, the instructions may specifically configure the processor to perform the algorithms and/or operations described herein when the instructions are executed. However, in some cases, the processor 30 may be a processor of a specific device (e.g., an electronic book or a mobile terminal) configured to employ an embodiment of the present innovation by further configuration of the processor by instructions for performing the algorithms and/or operations described herein. The processor 30 may include, among other things, a clock, an arithmetic logic unit (ALU) and logic gates configured to support operation of the processor.

In some embodiments, the apparatus 20 may include a user interface 34 that may, in turn, be in communication with the processor 30 to provide output to the user and to receive an indication of a user input. As such, the user interface 34 may include a display and, in some embodiments, may also include a keyboard, a mouse, a joystick, a touch screen, touch areas, soft keys, a microphone, a speaker, or other input/output mechanisms. Alternatively or additionally, the processor 30 may comprise user interface circuitry configured to control at least some functions of one or more user interface elements such as a display and, in some embodiments, a speaker, ringer, microphone and/or the like. The processor 30 and/or user interface circuitry comprising the processor may be configured to control one or more functions of one or more user interface elements through computer program instructions (e.g., software and/or firmware) stored on a memory accessible to the processor (e.g., memory device 32, and/or the like).

The apparatus 20 may optionally also include the communication interface 36, such as for communication with the database 22 or one or more health data collection devices 24. The communication interface 36 may be any means such as a device or circuitry embodied in either hardware or a combination of hardware and software that is configured to receive and/or transmit data from/to a network and/or any other device or module in communication with the apparatus 20. In this regard, the communication interface 36 may include, for example, an antenna (or multiple antennas) and supporting hardware and/or software for enabling communications with a wireless communication network. Additionally or alternatively, the communication interface 36 may include the circuitry for interacting with the antenna(s) to cause transmission of signals via the antenna(s) or to handle receipt of signals received via the antenna(s). In some environments, the communication interface 36 may alternatively or also support wired communication. As such, for example, the communication interface 36 may include a communication modem and/or other hardware/software for supporting communication via cable, digital subscriber line (DSL), universal serial bus (USB) or other mechanisms.

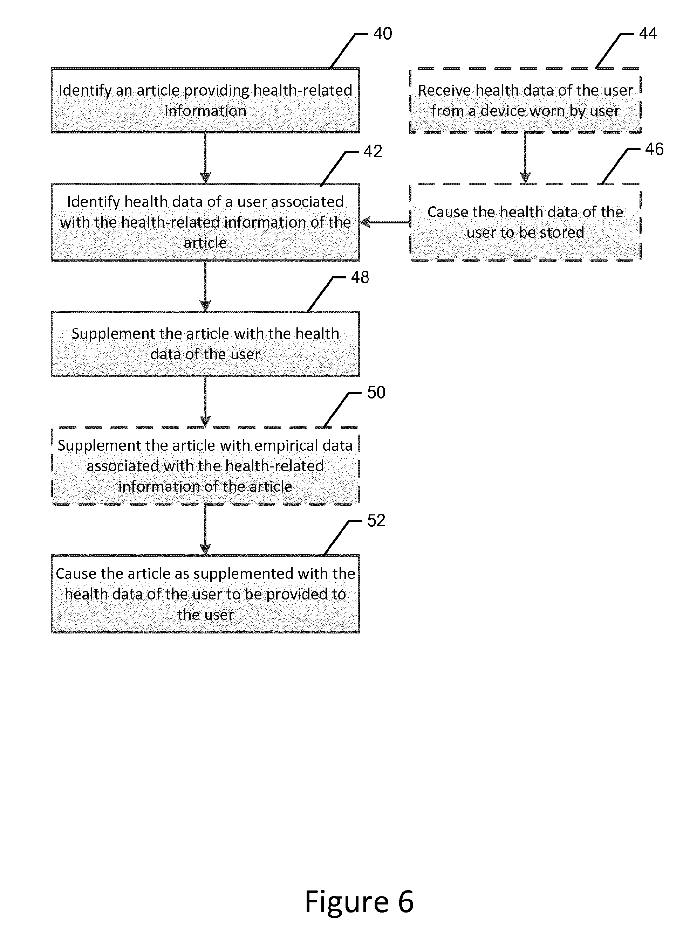

Referring now to Figure 6, the operations performed, such as by the apparatus 20 of an example embodiment, are depicted. The apparatus 20 includes means, such as the processor 30 or the like, for identifying an article 10 providing health-related information. See block 40 of Figure 6 with respect to the identification of an article 10. The health-related information may include any of a wide variety of information that effects the health of a person, either directly or indirectly. Additionally, the article 10 can include any of a variety of different types of content, such as textual information, graphical information or the like. The article 10 may be available electronically, such as by being stored in a database from which the article is searchable and retrievable. For example, the article 10 may be stored by the database 22, such as the article repository 22a of the embodiment of Figure 6, with which the apparatus 20 may in communication, such as via the communication interface 36. Alternatively, the article 10 may be available on line and may be identifiable by the apparatus 20 and accessible via the communication interface 36. Still further, the articles 10 may be stored locally, such as by memory 32 in some example embodiments. The article 10 may include any form of media including audio, video, virtual reality environments, etc. that is contextually relevant to the user and in which the health data 12 of the user may be presented.

In an example embodiment, the article 10 is identified to provide health-related information that is relevant to a health issue of the user or with which the user, a health care provider of the user, an insurance company associated with the user or the like is interested. In this example embodiment, the apparatus 20 includes means, such as the processor 30 or the like, for analyzing the health data 12of the user in order to detect a health issue of the user. In this regard, components of the apparatus 20, such as the processor 30, may be configured to analyze the health data collected by health data collection devices 24 and to identify a potential health issue, such as based upon a comparison of the health data 12 collected with respect to the user relative to predefined thresholds for comparable types of health data. Additionally or alternatively, the health data that is analyzed in order to detect a health issue of the user may include information provided by the user, a health care provider of the user, an insurance company associated with the user or the like that particularly identifies one or more health issues of the user or about which the user is interested. While a wide range of health issues may be detected based upon the health data 12 of the user, examples of health issues include diabetes, insomnia, lack of exercise or the like.

The apparatus 20 of this example embodiment also includes means, such as the processor 30 or the like, for identifying the article 10 based upon the health issue of the user such that the article provides health-related information relevant to the health issue. Thus, once a health issue of the user has been identified, the apparatus 20, such as the processor 30, may review the articles that are available in order to identify one or more articles 10 that are relevant to the particular health issue. The apparatus 20, such as the processor 30, may be configured to review the articles and to identify the relevant articles 10 in various manners, such as by a comparison of key words associated with the articles, such as may be maintained by metadata or otherwise embedded within the header of an article or within the article itself, which identify the topics with which the article is related to the health issues relevant to the user in order to identify articles that are related to the same health issue. Alternatively, the apparatus 20, such as the processor 30, may review the content of the articles, such as by conducting a text search of the articles or by scraping the article, to identify the context of the article and may thereafter compare the context of the article and the health issues of the user to determine if the article 10 is related to the health issues that are relevant to the user. Although the analysis discussed above relates to a component of the apparatus 20, such as the processor 30, performing the analysis, any combination of the analysis methods may be performed at a remote computing device and communicated to the apparatus 20.

The apparatus 20 of this example embodiment also includes means, such as the processor 30 or the like, for identifying health data 12 of the user that is associated with the health-related information of the article 10. See block 42 of Figure 6. In embodiments such as those described above in which the article 10 was identified in the first instance so as to be related to a health issue of the user, the apparatus 20, such as the processor 30, of this example embodiment may identify health data 12 of the user that is associated with the health-related information of the article by identifying the health data of the user that is related to the health issue that provided the context from which the article was identified.

In another embodiment, however, the apparatus 20, such as the processor 30, is configured to identify the health data by identifying the health data 12 of the user based upon data associated with the article 10 that at least partially defines the context of the article. For example, the data associated with the article 10 may include metadata associated with the article that defines the context of the article, such as by identifying one or more topics with which the article relates. Alternatively, the data associated with the article 10 that defines the context of the article may be identified by a review of the article, such as a text search of the article. In an example embodiment, the apparatus 20 of this example embodiment includes means, such as the processor 30 or the like, for scraping the article 10 to identify data within the article that at least partially defines the context of the article. Based upon the context of the article 10 as defined by the data associated with the article, the apparatus 20, such as the processor 30, may be configured to identify the health data 12 of the user that relates to or is otherwise relevant to the context of the article. For example, the article 10 may relate to the sleep patterns of millennials as illustrated in Figure 1 and the apparatus 20, such as the processor 30, may identify the health data 12 of the user that relates to the average amount of sleep that the user enjoys each day. Alternatively, the article 10 may relate to diseases or other conditions that are exacerbated by a lack of exercise with the health data 12 of the user that relates to the exercise patterns of the user being identified as a result of its correlation to the context of the article.

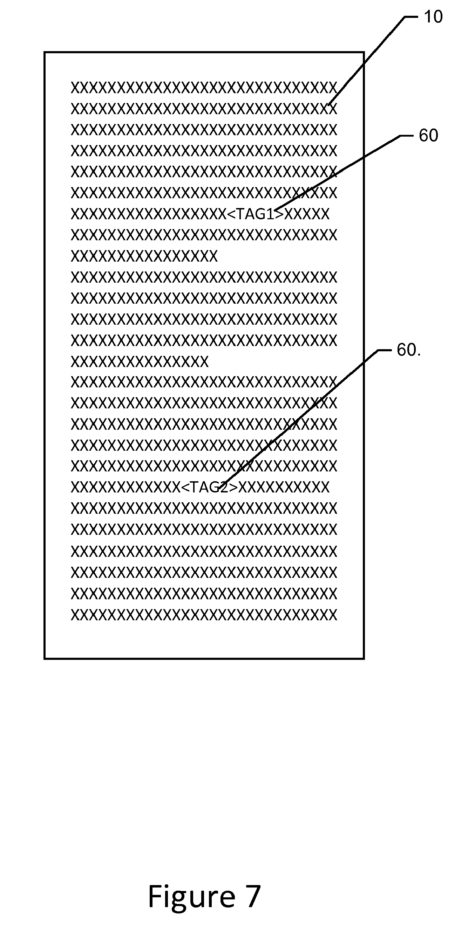

The apparatus 20, such as the processor 30, may be configured to identify health data 12 of a user that is associated with the health-related information of the article 10 in response to the identification of the article. However, in an example embodiment, the article 10 itself may include an indication that health data 12 of the user and/or empirical data 14 is to be accessed for presentation, for example, with the article. In one embodiment, the indication may be in the form of instructions, such as an application programming interface (API). In this example embodiment, once the article 10 has been identified, the apparatus 20, such as the processor 30, may be configured to execute the instructions included within the article which, in turn, causes the health data 12 of the user to be identified. For example, the execution of the instruction may create an API call that, in turn, identifies the health data 12 of the user that is relevant to the health-related information of the article 10. Additionally or alternatively, as shown in Figure 7, the article 10 may include tags 60, such as metadata tags, that serve as indications that health data 12 of the user and/or empirical data 14 is to be accessed. As such, the apparatus 20, such as the processor 30, may recognize the tags 60 and, in turn, identify the health data 12 of the user and/or empirical data 14 that is to be presented within the article 10 at the location identified by the tag. While API calls and metadata tags 60 are described herein, the article 10 may include other forms of indication that cause the apparatus 20, such as the processor 30, to identify the health data 12 of the user and/or empirical data 14 that is to be presented within the article.

As described above, the health data 12, such as biometric data, of the user may be provided in various manners and may be stored, for example, in memory 32 or in database 22. In an example embodiment, however, the apparatus 20 includes means, such as the processor 30, the user interface 34, the communication interface 36 or the like, for receiving health data 12 of the user from a health data collection device 24 worn by the user. See block 50 of Figure 6. In this example embodiment, the apparatus 20 also includes means, such as the processor 30, the memory 32 or the like, for causing the health data 12 of the user to be stored as shown in block 52. As such, the apparatus 20, such as the processor 30, of this example embodiment identifies the health data 12 of the user that is associated with the health-related information of an article 10 by reviewing the health data of the user that is received from the health data collection device 24.

The apparatus 20 of an example embodiment also includes means, such as the processor 30 or the like, for supplementing the article 10 with the health data 12 of the user. See block 44 of Figure 6. In relation to supplementation of the article 10 with the health data 12 of the user, the health data itself may be incorporated within the article and then provided, e.g., presented, in association with the article. Alternatively, a representation of the health data 12 may be provided so as to supplement the article 10, such as by providing an average of the health data or representation that characterizes the health data within or in association with the article. As shown in Figure 1, for example, health data 12 of the user that is relevant to the health-related information of the article 10 may be included or embedded within the article. The health data 12 with which the article 10 is supplemented may be presented in various manners including numerically, textually or graphically, such as the bar graph of Figure 1 which illustrates the average night's sleep of the user. In order to supplement the article 10 with the health data 12 of the user, the apparatus 20 of an example embodiment include means, such as the processor 30 or the like, for creating space within the article in which the health data is inserted without eliminating or replacing, partially replacing, wholly replacing, providng in a separate window, or any combination thereof any content otherwise provided by the article. As shown in Figure 1, for example, a space 16 may be created in the article 10 in which to insert the graphical representation of the health data 12 of the user, thereby repositioning, but not replacing any of the content of the article.

The apparatus 20 of an example embodiment also includes means, such as the processor 30, the user interface 34 or the like, for causing the article 10 as supplemented with the health data 12 of the user to be provided to the user. See block 48 of Figure 6. In this regard, the article 10 may be provided for display, such as via the user interface 34 in a manner that facilitates the user's review of both the content of the article and the relevant health data 12 of the user. See, for example, Figures 1 and 2. As such, the article 10 may provide an educational benefit to the user in relation to the health-related information conveyed thereby. By including the actual health data 12 of the user that is relevant to the health-related information of the article 10, the interest of the user in the article may be enhanced and the user may be able to view their own health data in context to the health-related information that is otherwise described in a related sense by the article. Thereafter, the user may be more informed and, in some instances, may make behavioral or other lifestyle-related changes in order to address the health issues with which the health data 12 of the user is related, such as in instances in which the health data of the user fails to satisfy the norms or the recommended behavior defined by the health-related information provided by the article 10.

In order to provide additional information to the user related to the health-related information of the article 10, the apparatus 20 of an example embodiment also includes means, such as the processor 30 or the like, for additionally supplementing the article with empirical data 14 associated with the health-related information of the article. See block 46 of Figure 6. The empirical data 14 may be any of a wide variety of data related to the health-related information of the article 10. In an example embodiment, however, the empirical data 14 is data of the same type as the health data 12 of the user, but collected from a larger population. For example, in instances in which the article 10 relates to diabetes and the health data 12 of the user relates to blood glucose readings of the user, the empirical data 14 may also relate to blood glucose readings collected from a larger population sample and presented, for example, in the aggregate, such as an average. As another example in which the article 10 relates to average amounts of sleep and the health data 12 of the user relates to the user's average amounts of sleep, the empirical data 14 may also relate to an average amount of sleep for a larger sample of the population.

The empirical data 14 may be obtained in various manners. For example, the empirical data 14 may be obtained, such as by the processor 30, from the article 10. Alternatively, the empirical data 10 may be obtained from the database 22, such as a government database, a peer review database, a private database, such as a database maintained by a health care provider or an insurance company, or other empirical data repository 22b as shown in Figure 6. As with the health data 12, the empirical data 14 may be presented in various manners including numerically, textually or graphically. In an example embodiment in which the article 10 is also supplemented with empirical data 14, the apparatus 20, such as the processor 30, may be configured to supplement the article with the health data 12 of the user and with empirical data by providing a comparison between the health data of the user and the empirical data within the article. As shown in Figure 1, the article 10 may be supplemented with both health data of the user 12 and empirical data 14 that are presented graphically, such as a bar graph. Based upon the review of the article 10, the health data 12 of the user and the empirical data 14, a user may be able to view their own health data in better context, such as in terms of the context provided by the health-related information of the article and relative to the empirical data with which the article supplemented. Thus, the user may make better informed choices going forward in order to address the health issue with which the health data 12 is related based upon the user's review of their health data in context.

The apparatus 20, method and computer program product of an example embodiment provide numerous technical advantages. For example, the apparatus 20, method and computer program product of an example embodiment identify the context of an article 10 and then identify contextually-relevant health data 12 of a user and/or empirical data 14 in an efficient manner for presentation in an integrated manner to the user. By identifying the context of an article 10 and then identifying contextually-relevant health data 12 of a user and/or empirical data 14 in an efficient manner, the apparatus 20, method and computer program product of an example embodiment provide useful information to the user while conserving processing resources and reducing power consumption, particularly relative to a system that is responsive to searches generated by a user in an effort to identify their health data and/or empirical data that is relevant to an article read by the user.

As described above, Figure 6 illustrates a flowchart of an apparatus 20, method, and computer program product according to example embodiments of the innovation. It will be understood that each block of the flowchart, and combinations of blocks in the flowchart, may be implemented by various means, such as hardware, firmware, processor, circuitry, and/or other devices associated with execution of software including one or more computer program instructions. For example, one or more of the procedures described above may be embodied by computer program instructions. In this regard, the computer program instructions which embody the procedures described above may be stored by the memory device 32 of an apparatus 20 employing an embodiment of the present innovation and executed by the processor 30 of the apparatus. As will be appreciated, any such computer program instructions may be loaded onto a computer or other programmable apparatus (e.g., hardware) to produce a machine, such that the resulting computer or other programmable apparatus implements the functions specified in the flowchart blocks. These computer program instructions may also be stored in a computer-readable memory that may direct a computer or other programmable apparatus to function in a particular manner, such that the instructions stored in the computer-readable memory produce an article of manufacture the execution of which implements the function specified in the flowchart blocks. The computer program instructions may also be loaded onto a computer or other programmable apparatus to cause a series of operations to be performed on the computer or other programmable apparatus to produce a computer-implemented process such that the instructions which execute on the computer or other programmable apparatus provide operations for implementing the functions specified in the flowchart blocks.

Accordingly, blocks of the flowchart support combinations of means for performing the specified functions and combinations of operations for performing the specified functions for performing the specified functions. It will also be understood that one or more blocks of the flowchart, and combinations of blocks in the flowchart, can be implemented by special purpose hardware-based computer systems which perform the specified functions, or combinations of special purpose hardware and computer instructions.

In some embodiments, certain ones of the operations above may be modified or further amplified. Furthermore, in some embodiments, additional optional operations may be included. Modifications, additions, or amplifications to the operations above may be performed in any order and in any combination.

Many modifications and other embodiments of the innovations set forth herein will come to mind to one skilled in the art to which these innovations pertain having the benefit of the teachings presented in the foregoing descriptions and the associated drawings. Therefore, it is to be understood that the innovations are not to be limited to the specific embodiments disclosed and that modifications and other embodiments are intended to be included within the scope of the appended claims. Moreover, although the foregoing descriptions and the associated drawings describe example embodiments in the context of certain example combinations of elements and/or functions, it should be appreciated that different combinations of elements and/or functions may be provided by alternative embodiments without departing from the scope of the appended claims. In this regard, for example, different combinations of elements and/or functions than those explicitly described above are also contemplated as may be set forth in some of the appended claims. Although specific terms are employed herein, they are used in a generic and descriptive sense only and not for purposes of limitation.

## Claims

1. A method comprising:
identifying an article providing health-related information;
identifying health data of a user that is associated with the health-related information of the article;
supplementing the article with the health data of the user; and
causing the article as supplemented with the health data of the user to be provided to the user.

2. A method according to Claim 1 wherein identifying health data comprises identifying health data of the user based upon data associated with the article that at least partially defines a context of the article.

3. A method according to Claim 2 wherein the data associated with the article comprises metadata associated with the article.

4. A method according to Claim 2 wherein identifying health data of the user based upon data associated with the article comprises scraping the article to identify data within the article that at least partially defines a context of the article.

5. A method according to any one of Claims 1 to 4 wherein identifying health data comprises identifying the health data of the user in response to execution of an instruction included within the article.

6. A method according to any one of Claims 1 to 5 further comprising:
receiving health data of the user from a device worn by the user; and
causing the health data of the user to be stored.

7. A method according to any one of Claims 1 to 6 further comprising additionally supplementing the article with empirical data associated with the health-related information of the article.

8. A method according to Claim 7 wherein supplementing the article with the health data of the user and with empirical data comprises providing a comparison between the health data of the user and the empirical data within the article.

9. A method according to Claim 7 further comprising obtaining the empirical data from the article.

10. A method according to any one of Claims 1 to 9 wherein identifying an article comprises:
analyzing the health data of the user in order to detect a health issue of the user; and
identifying the article based upon the health issue of the user such that the article provides health-related information relevant to the health issue.

11. A method according to any one of Claims 1 to 10 wherein supplementing the article with the health data of the user comprises creating space within the article in which the health data is inserted without eliminating any content associated with the article.

12. An apparatus comprising at least one processor and at least one memory including computer program code, the at least one memory and the computer program code configured to, with the processor, cause the apparatus to at least:
identify an article providing health-related information;
identify health data of a user that is associated with the health-related information of the article;
supplement the article with the health data of the user; and
cause the article as supplemented with the health data of the user to be provided to the user.

13. The apparatus according to claim 12, wherein the at least one memory and the computer program code are further configured to cause the apparatus to perform the method of any of Claims 1 to 11.

14. A computer program product comprising at least one non-transitory computer-readable storage medium having computer-executable program code instructions stored therein, the computer-executable program code instructions comprising program code instructions to at least:
identify an article providing health-related information;
identify health data of a user that is associated with the health-related information of the article;
supplement the article with the health data of the user; and
cause the article as supplemented with the health data of the user to be provided to the user.

15. The computer program product according to claim 14, wherein the computer-executable program code instructions further comprise program code instructions to perform the method of any of Claims 1 to 11.
